# EUROPEAN PATENT SPECIFICATION

(11) **EP 3 850 636 B1**
(45) Date of publication and mention of the grant of the patent: **03.04.2024**
(21) Application number: 19783744.6
(22) Date of filing: 25.09.2019
(51) Int. Cl.: G16H 40/20

(54) **AN OPERATING ROOM CONTROL SYSTEM, METHOD, AND PROGRAM**
EIN OP-STEUERUNGSSYSTEM, -VERFAHREN UND -PROGRAMM
SYSTEME, PROCÉDÉ ET PROGRAMME DE COMMANDE DE SALLE OPÉRATOIRE

(30) Priority: 12.10.2018 EP 18200257
(43) Date of publication of application: 21.07.2021
(73) Proprietor: Sony Group Corporation, Tokyo 108-0075 (JP)
(72) Inventor: WRIGHT, Christopher, London, SE193HF (GB); KAMODA, Akinori, Tokyo 108-0075 (JP)
(74) Representative: D Young & Co LLP
(86) International application number: PCT/JP2019/037639
(87) International publication number: WO 2020/075502

(56) References cited:
- WO-A1-2017/214696
- WO-A1-2018/134140
- US-A1- 2003 076 293
- US-A1- 2009 300 507

## Description

### Technical Field

The present disclosure relates to an operating room control system, method and computer program.

### Background

The "background" description provided herein is for the purpose of generally presenting the context of the disclosure. Work of the presently named inventors, to the extent it is described in the background section, as well as aspects of the description which may not otherwise qualify as prior art at the time of filing, are neither expressly or impliedly admitted as prior art against the present disclosure.

With a constantly expanding range and availability of tools and equipment, modern preoperative surgical setup has become an increasingly complex and time-consuming process. The introduction of new technologies, while a welcome benefit to patients and medical professionals alike, cause further integration issues. Turnover time between subsequent operations (depending on the operation type) can range from averages of approximately 30 to 50 minutes or more, and significant delays can often result from unavailable instruments or supplies (in 10% of operations) and technology issues (in 7% of operations).

Surgical delays (resulting from a range of issues including room and equipment setup problems) can also affect the performance of the surgeon and support staff by causing increased anxiety and frustration, and delays in one surgery cause a domino effect that impacts on all subsequent surgeries over the course of the working day.

Systems have previously been developed that attempt to reduce the turnover time between operations, by using techniques such as Six Sigma analysis. However, these processes do not account for the increasing technical difficulty of setting up the operating room devices, focusing more on the availability of technicians and instrument scheduling clashes. In other words, these previously developed systems use process engineering to reduce turnover time between operations.

Other previous systems include Workflow Simulator by Siemens (registered trademark) which simulate surgical workflows and strategic planning in healthcare. This system offers an exact replica of the real surgical process, including turnover between operations, and allows new processes and technology integrations to be tested before they are implemented in the real operating room.

However, none of these previous systems reduce the operating room setup times by account of individual user preferences (such as surgeon preferences) and the dimensions of individual operating theatres.

Further, none of these previous systems simplify device usage in the operating room. This reduces mid-surgery operating room delays and may reduce communication interference in the operating theatre.

It is an aim of the present disclosure to address at least one of these issues.

Prior art Includes:

WO 2017/214696, which relates to navigation systems to track objects in image-guided medical procedures;

WO 2018/134140 discloses systems and methods for performing augmented reality interventional medical procedures;

US 2003/076293 discloses a gesture recognition system which does not require complex computing resources;

US 2009/300507 addresses a system for automatically determining the presence of devices within a medical room.

### SUMMARY

Embodiments of the present disclosure are defined by the appended claims.

The foregoing paragraphs have been provided by way of general introduction, and are not intended to limit the scope of the following claims. The described embodiments, together with further advantages, will be best understood by reference to the following detailed description taken in conjunction with the accompanying drawings.

### BRIEF DESCRIPTION OF THE DRAWINGS

A more complete appreciation of the disclosure and many of the attendant advantages thereof will be readily obtained as the same becomes better understood by reference to the following detailed description when considered in connection with the accompanying drawings, wherein:
Figure 1 shows a plan view of an operating room 100 according to embodiments of the present disclosure;
Figure 2 shows a block diagram of a room control device 200 according to embodiments of the disclosure;
Figure 3 shows a surgical device apparatus 300 according to embodiments of the disclosure;
Figure 4 shows a display device 400 according to embodiments of the disclosure;
Figure 5 shows an example of the operating room 100 with the defined regions;
Figure 6 shows a flow chart according to embodiments of the disclosure; and
Figures 7A and 7B show an example of at least part of an operating room with the defined regions.

### DESCRIPTION OF THE EMBODIMENTS

Referring now to the drawings, wherein like reference numerals designate identical or corresponding parts throughout the several views.

### Description of Embodiments

Referring now to the drawings, wherein like reference numerals designate identical or corresponding parts throughout the several views.

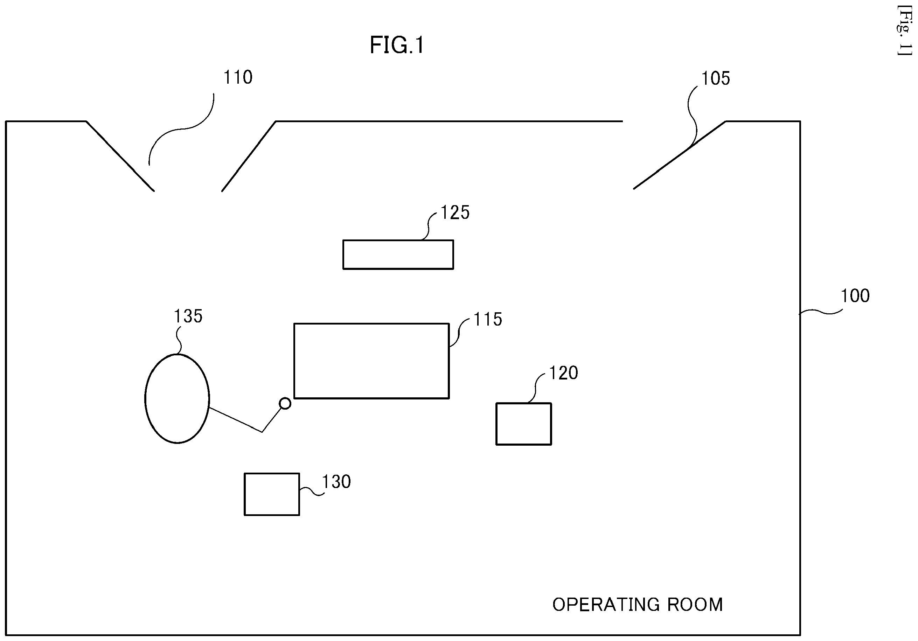

FIG. 1 shows a plan view of an operating room 100 according to embodiments of the present disclosure. The operating room 100 has a staff door 105 through which members of the surgical team enter. The operating room 100 also has a patient door 110 through which the patient enters. Typically, the patient will enter the operating room 100 on a surgical table which is wheeled into position. The surgical table 115 is typically positioned under a surgical light (not shown). Additionally provided in the operating room 100 are surgical devices. In the example embodiment of FIG. 1, a surgical microscope 135 is shown. The surgical microscope 135 has a base and an extending arm upon which the microscope head is provided. The surgical microscope 135 is typically a large piece of surgical equipment that is positioned close to the patient and the surgeon. Whilst it is typical that the arm of the surgical microscope 135 has a high level of mobility, the surgical microscope 135 is typically difficult to move.

A surgical trolley 130 is also shown. The surgical trolley 130 has a number of surgical instruments placed thereon. For example, the surgical trolley 130 has cutting and dissecting instruments such as scalpels and scissors, and grasping or holding instruments such as forceps, towel clamps and the like. Other instruments such as surgical drills may be placed on the surgical trolley 130.

An anaesthetist station 120 is also shown. The anaesthetist station 120 includes an anaesthetist and associated equipment such as an anaesthesia machine, tracheostomy tube, Tuohy needle, oxygen mask and the like.

A monitor bank 125 is shown. The monitor bank 125 typically displays a visual output from many of the surgical devices in the operating room 100. The monitor bank 125 may therefore comprise a plurality of independent screens, each screen connected to a different surgical device, or may be a large screen having independent areas of the screen dedicated to each output. The layout of the surgical device output will depend upon the surgical devices used in the surgical procedure and the personal preferences of the surgeon. This means that set up of the monitor bank 125 may be quite complicated using known techniques and is very difficult to change during the surgical procedure.

As will be appreciated, setting up an operating room 100 such as that shown in FIG. 1 is complicated. This is especially the case given that each surgical device may be used in different surgical procedures and the location and the settings associated with each procedure are different. For example, the surgical microscope 135 may be located near the patient's head for a Neurosurgery procedure such as a craniotomy and will be located near the patient's legs for a leg surgical procedure such as Meniscectomy. Given the size of the surgical microscope, this can significantly impact the position of other surgical devices within the operating room 100 during setup.

Further, the settings required for a neurosurgical procedure and a leg surgical procedure may be very different. This is further complicated since different surgeons have different preferred settings. This makes ensuring that surgical devices are correctly setup very difficult and time consuming.

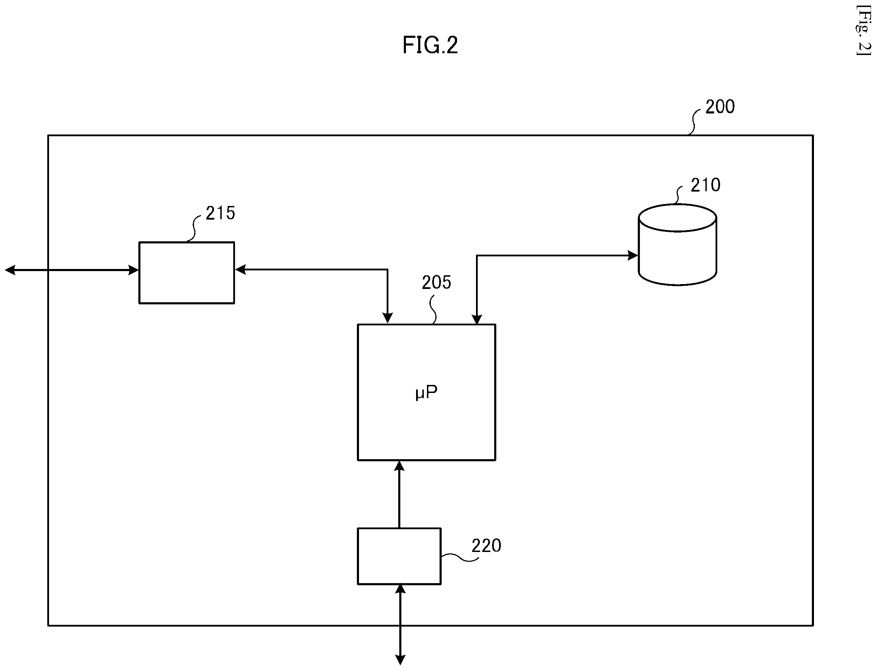

FIG. 2 shows a block diagram of a room control device 200 according to embodiments of the disclosure. The room control device 200 aims to address the problems identified above. The room control device 200 includes a control device processor 205. The control device processor 205 is typically embodied as processor circuitry such as a microprocessor which is configured to operate using computer readable code. The control device processor 205 controls the operation of the room control device 200 using the computer readable code. Of course, the control device processor 205 may be embodied as hardware (such as an Application Specific Integrated Circuit, Field Programmable Gate Array or the like).

Additionally connected to the control device processor 205 is control device storage 210. The control device storage 210 is a computer readable storage medium (such as an optically readable, magnetically readable or solid state). The control device storage 210 is configured to store the computer readable code using which the control device processor 205 operates. In addition, user profiles and various data structures are stored in the control device storage 210. In embodiments, the data structures may include one or more databases for different surgical procedures, different surgical devices or different surgeons as will be explained later.

Additionally connected to the control device processor 205 is control device communication circuitry 215. The control device communication circuitry 215 is configured to communicate with other devices which may be located within the operating room 100. This communication may be over a wired network (such as an Ethernet network) or may be over a wireless network (such as a WiFi network). The purpose of the control device communication circuitry 215 is to provide data to and receive data from the other devices within the operating room 100 as will become apparent. Moreover, the control device communication circuitry 215 may communicate with a centralised data system of a medical facility where information relating to surgical procedure timetables, surgeon profiles and preferences, operating room dimensions and layout or surgical device parameters may be stored.

Finally, control device display circuitry 220 is connected to the control device processor 205. The control device display circuitry 220 is configured to display, to a user, information that is used to define various physical locations within the room. As will become apparent, the provision of the various physical locations within the room assists in addressing the aforesaid problems. The control device display circuitry 220 may display the information to a user by projecting one or more lines into the room. Alternatively or additionally, the control device display circuitry 220 may interact with an Augmented Reality (AR) system or a Virtual Reality (VR) system worn by an operating room technician when setting up the operating room 100 or a member of the surgical team, such as a surgeon, when performing the surgery to project the one or more lines onto the operating room 100 viewed through the AR or VR system.

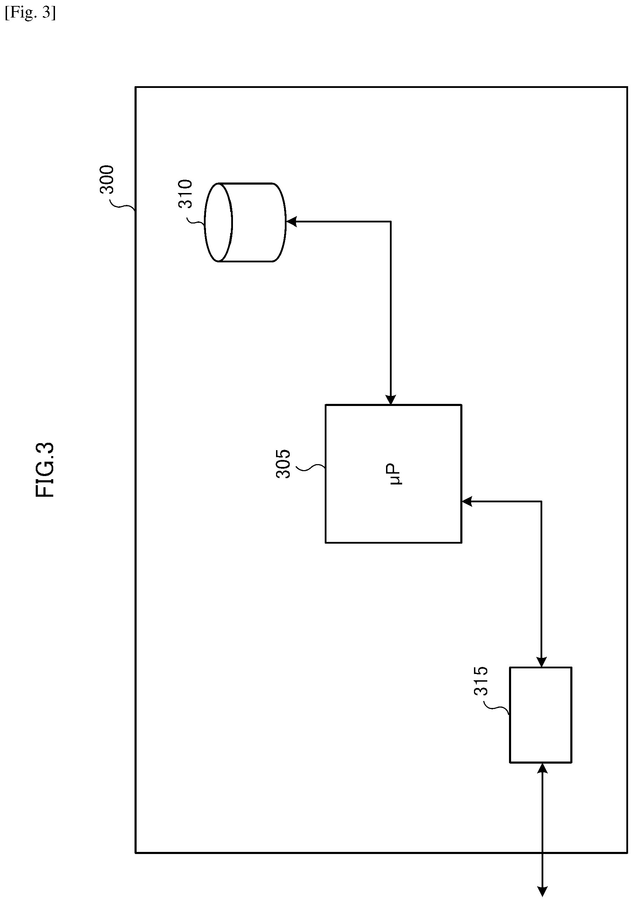

Referring to FIG. 3, a surgical device apparatus 300 according to embodiments is shown. The surgical device apparatus 300 will be coupled to, or be integrated with, a surgical device used in surgery. For example, the surgical device apparatus 300 may be coupled to or integrated with the surgical microscope 135, the surgical trolley 130, the anaesthetist station 120, the monitor bank 125, a surgical drill, endoscope or any medical apparatus or the like. Of course, the disclosure is not so limited and the surgical device apparatus 300 may be a stand-alone device.

The surgical device apparatus 300 includes a surgical device processor 305. In a similar manner to the control device processor 205, the surgical device processor 305 is typically embodied as processor circuitry such as a microprocessor which is configured to operate using computer readable code. The surgical device processor 305 controls the operation of the surgical device apparatus 300 using the computer readable code. Of course, the surgical device processor 305 may be embodied as hardware (such as an Application Specific Integrated Circuit, Field Programmable Gate Array or the like).

Additionally connected to the surgical device processor 305 is surgical device storage 310. The surgical device storage 310 is a computer readable storage medium (such as an optically readable, magnetically readable or solid state). The surgical device storage 310 is configured to store the computer readable code using which the surgical device processor 305 operates. The surgical device storage 310 also includes an identifier that identifies the surgical device apparatus 300 (either uniquely or non-uniquely) and, where appropriate, surgical device settings such as brightness and contrast settings where a display is provided on the surgical device or other settings such as drill speed settings where the surgical device is a drill.

Additionally connected to the surgical device processor 305 is surgical device communication circuitry 315. The surgical device communication circuitry 315 is configured to the room control device 200 and, in embodiments, other surgical devices within the operating room 100. This communication may be over a wired network (such as an Ethernet network) or may be over a wireless network (such as a WiFi network). The purpose of the surgical device communication circuitry 315 is to provide data to and receive data from the room control device 200 other possibly other devices within the operating room 100 as will become apparent.

There is also optionally provided surgical device location circuitry 320 connected to the surgical device processor 305. In this case, the surgical device location circuitry 320 may use image recognition techniques which use an image of the operating room captured by one or more camera located within the operating room, WiFi device location techniques to identify the location of the surgical device. Of course, the disclosure is not so limited and other techniques for identifying the location of the surgical device are envisaged such as Near Field Communication (NFC) which may include the One Touch Smart Mat or the like.

The accuracy and/or scale of operation of the surgical device location circuitry 320 will depend upon the size of the regions located in the room as will become apparent later. Moreover, the surgical device location circuitry 320 may operate in either two dimensions or three dimensions.

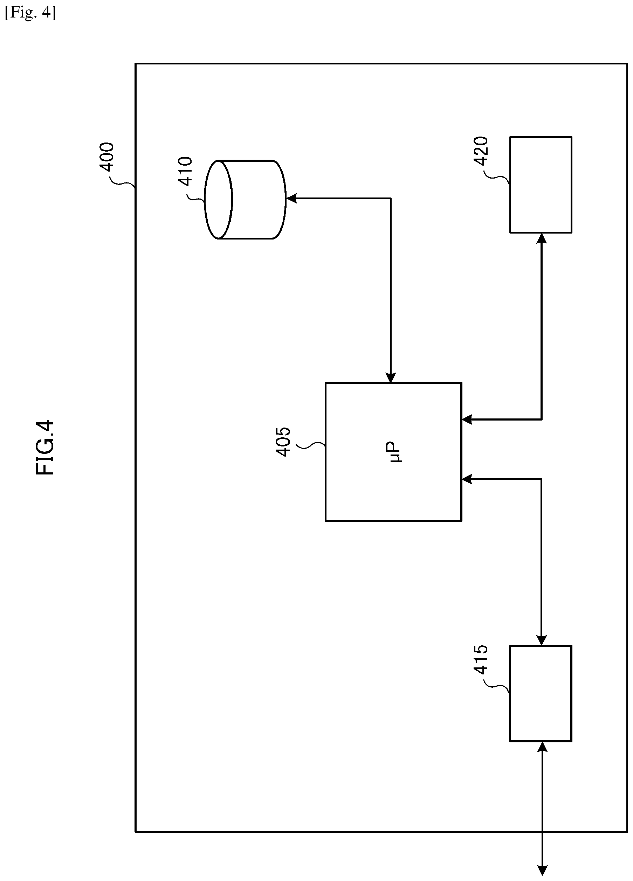

Referring to FIG. 4, a display device 400 according to embodiments is shown. The display device 400 includes a display device processor 405. In a similar manner to the control device processor 205, the display device processor 405 is typically embodied as processor circuitry such as a microprocessor which is configured to operate using computer readable code. The display device processor 405 controls the operation of the display device 400 using the computer readable code. Of course, the surgical device processor 305 may be embodied as hardware (such as an Application Specific Integrated Circuit, Field Programmable Gate Array or the like).

Additionally connected to the display device processor 405 is display device storage 410. The display device storage 410 is a computer readable storage medium (such as an optically readable, magnetically readable or solid state). The display device storage 410 is configured to store the computer readable code using which the display device processor 405 operates.

Additionally connected to the display device processor 405 is display device communication circuitry 415. The display device communication circuitry 415 is configured to communicate with the room control device 200. This communication may be over a wired network (such as an Ethernet network) or may be over a wireless network (such as a WiFi network). The purpose of the display device communication circuitry 415 is to provide data to and receive data from the room control device 200.

It is envisaged that the display device 400 may be attached to or form part of a projection device (such as a laser projector) to project lines into the operating room 100 or may be attached to or form part of an AR or VR headset to be worn by an operating room technician or a member of the surgical team.

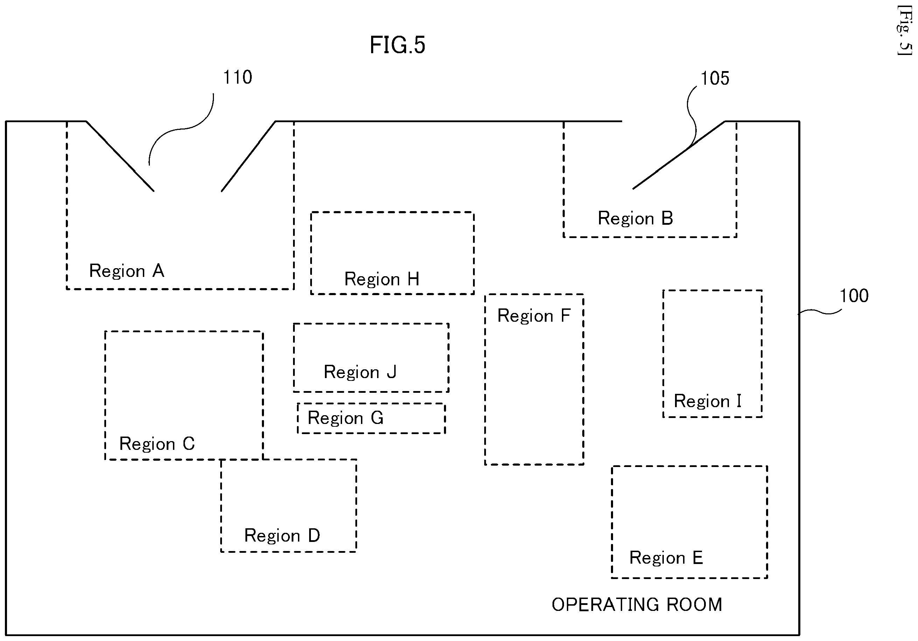

FIG. 5 shows an operating room 100 of FIG. 1 according to embodiments of the disclosure. Specifically, FIG. 5A shows the operating room 100 of FIG. 1 with various physical regions defined within the operating theatre when viewed using the display device 400.

In FIG. 5, various regions within the operating room 100 are defined. As will be explained later, each region has a predefined surgical device attribute defining a characteristic of one or more surgical device located within that region. In other words, the operating room 100 is divided into a plurality of regions and within at least one of the regions, a different surgical device attribute is allocated to that region. In embodiments, the surgical device attribute may be that a specific surgical device or type of surgical device is to be placed in that region. This means that when the user is an operating room technician, the technician will know in which regions to place the specific surgical device or type of surgical device.

In order to enable the technician to know in which regions to place the surgical device, the control device display circuitry 220 provides an image to the user (via the display device 400) where the plurality of regions are overlaid on the operating room. In other words, the plurality of regions may be overlaid on the actual operating room using the display device 400 embodied as a projector or are overlaid on eyewear when using the display device embodied as an AR or a VR device. This arrangement allows the operating room technician to place the surgical devices in their correct location within the operating room more quickly.

After placing the surgical devices in their correct location, the room control device 200 may verify that the surgical devices are located in the correct location. This reduces the likelihood of an error in placement of the surgical device which may lead to complications once the surgery has started.

In order to verify that the surgical devices have been correctly positioned, the room control device 200 will need to identify the location of each surgical device. Of course, although the following describes identifying the location of each surgical device, the disclosure is not so limited. In particular, only the location of one or more surgical devices may be verified. For example, only the location of surgical devices with limited mobility (such as a surgical microscope) may be verified.

The room control device 200 may identify the location of the surgical device using any appropriate technique. For example, the room control device 200 may receive positional information (such as a co-ordinate of a position within the room) from the surgical device itself or may establish the location using image recognition techniques.

In embodiments where the room control device 200 receives positional information, the surgical device may optionally include a surgical device apparatus 300. In this case, the location and identity of the surgical device apparatus 300 will be communicated to the room control device 200. The room control device 200 will then compare the actual location and identity of the surgical device with the desired location and identity of the surgical device. In the event that the surgical device is placed within the correct region, or is within a threshold distance of the correct region, the room control device 200 will confirm that the surgical device has been placed in the correct location. In the event that the surgical device is not placed within the correct region, or is outside of the threshold distance, the room control device 200 will confirm that the surgical device has been placed in the incorrect location. This ensures that the surgical device is positioned in the correct location within the operating room 100.

Of course, the disclosure is not so limited. It is possible that the control device 200 captures an image of the operating theatre 100 and identifies the position of each surgical device within the operating theatre 100. This may be achieved by using image recognition or may be achieved by capturing an identifier (such as a QR code) or light emitting identifier located on the surgical device which uniquely identifies the surgical device.

In either case above, the surgical device attribute may include specific surgical device settings to be applied to a particular surgical device. In other words, in embodiments, a surgical device located within that region will have predefined device settings applied to it. Alternatively, the surgical device attribute may be the positional information of the surgical device. This can be sent to the room control device 200 to verify the correct placement of the surgical device in the region.

Additionally, it is envisaged that when a surgical device is located within that region, predefined device settings may be applied to other devices within the operating room 100. These other devices may be other surgical devices within the operating room 100 or may be infrastructure within the operating room 100, or other, non-surgical devices. For example, when a surgical device that is sensitive to electromagnetic interference (such as a ventilator) is located within a particular location, the other surgical devices have any transmitting functions disabled. The transmitting functions may be disabled for the entirety of the surgery or only when the sensitive surgical device is operational. For non-surgical devices, such as mobile phones, an application may be installed on the mobile telephone that communicates with the control device 200 and allows the control device 200 to disable the transmitting function within the mobile phone.

In a slight modification of this embodiment, areas within the operating room may be defined wherein when a surgical device is placed in the area, the transmitting function is disabled. In other words, the predefined device characteristics may be to disable the wireless communication function. This is termed a "Transmission Off Zone". In the example of FIG. 5A below, Region E is a Transmission Off zone.

It should be noted that the room control device 200 may disable the wireless communication function of the surgical device directly or using a different device such as using a router to disable the wireless communication function of the surgical device.

It should be noted that the disabling of the communication function of the surgical device is not limited to the wireless communication function, namely, the room control device 200 may disable the wired-communication function of the surgical device directly or using a different device such as using a router to disable the wired-communication function of the surgical device.

### «Example Embodiments of Application of Predefined Device Settings»

### <Example 1>

Fluorescence imaging is being used during a surgical procedure to detect a tumour in the patient. The room control device 200 has stored an association that when fluorescence imaging is used, it is necessary to dim the lights in the operating room to achieve an improved signal-to-noise ratio.

During setup, the control device 200 defines a location within a section of a surgical trolley where the fluorescence imaging device will be stored. When the fluorescence imaging device is located in the section of the surgical trolley, the control device 200 applies predefined parameters to the surgical device. In this case, the control device 200 also applies a setting to infrastructure within the operating room 100. Specifically, the control device 200 controls the brightness of the lights in the room, depending on whether the device is located within the defined location.

The surgery begins, and eventually the surgeon needs to use the fluorescence imaging device and picks it up from the trolley. At this point, the control device 200 identifies that the fluorescence imaging device has been picked up from the trolley using the location information for the fluorescence imaging device.

The control device 200 detects that the fluorescence imaging device has been removed from its defined location and then dims the lights in the operating room to an appropriate level.

When the surgeon is finished using the fluorescence imaging device, the surgeon returns it to the trolley. The control device 200 detects that the fluorescence imaging device has been returned to its defined location and returns the lights to a normal brightness level.

### <Example 2>

During a surgical procedure, a senior surgeon is working with a trainee surgeon.

The senior surgeon is teaching the junior surgeon how to use a surgical drill.

The surgical drill has a plurality of defined regions and parameters that are to be applied to the surgical drill when placed in each of the defined regions. Specifically, when the surgical drill is placed in one of the defined regions, the settings applied to the surgical drill are appropriate for the trainee surgeon and when the surgical drill is placed in a second of the defined regions, the settings are appropriate for the senior surgeon. Typically, the maximum speed of the surgical drill is lower for the trainee surgeon. For example, when drilling a femur for a pin, the maximum speed for the drill when used by the senior surgeon is 1500 rpm and when used by the trainee surgeon is 800 rpm. The lower speed setting for use by the trainee surgeon reduces any damage from a mistake made by the trainee surgeon and allows the senior surgeon to more easily monitor the trainee's work.

By changing the settings based on the region of placement of the surgical device allows the device settings to be easily changed and reduces the likelihood of an erroneous setting being applied to the surgical device.

Although the above describes changing the surgical device setting depending upon the region in which the surgical device is placed, the disclosure is not so limited. For example, machine vision techniques, such as face recognition, may be used to identify surgeons individually and apply the device settings depending on the user. Surgical staff profiles may be used to identify experienced and inexperienced surgeons.

### <Example 3>

In this example, the operating room 100 has three different monitor screens being set up for a surgical procedure involving two surgeons and an ultrasonographer.

A technician brings in a trolley with a connected diagnostic ultrasound control box into the room.

Three adjacent regions are created based on the predicted ultrasonographer position in the room. The control device 200 labels the regions and displays the label to the technician. Specifically, the labels are:
∘ Monitor 1
∘ Monitor 2
∘ Monitor 1+2

The technician places the trolley in the first region (labelled "Monitor 1"). The device parameter (monitor configuration) associated with the first region is to connect the surgical device to monitor 1. This monitor may be located nearest to the ultrasonographer's station.

During the procedure, the ultrasonographer changes position. As he does this he moves the ultrasonography trolley a small distance into the second region (labelled "Monitor 2"). The device parameter associated with the second region is to connect the surgical device to monitor 2. The ultrasound image is switched from the first monitor to the second monitor.

If the ultrasonographer moves the trolley into the third region (labelled "Monitor 1+2"), the device parameter associated with the third region is to connect the surgical device to both the first monitor and the second monitor.

This allows the members of the surgical team to easily change the operating parameters of the surgical device.

### <Example 4>

A surgeon picks up an ultrasound device during an emergency procedure which has not had significant pre-planning time.

Three regions are created where the parameters associated with each region correspond to different optimal imaging settings for different tissue types. The boundaries of the regions are defined near to the patient's heart, lungs and abdomen, respectively.

These regions are projected onto the drape covering the patient (either being projected directly onto the drape or being projected onto an AR display worn by the surgeon). The surgeon selects the cardiac imaging parameters by placing the ultrasound tool in the region located near to the patient's heart. The surgeon proceeds to image the patient's heart with the optimal settings.

More generally, the image setting of the surgical device is set in dependence upon the tissue of the patient. This is further exemplified with spectrum imaging for acquiring different spectrum of the different tissue or organs being imaged. For example, the spectrum provided by heart tissue is different to brain tissue or bone.

### <Regions>

In the embodiments described above, the various regions are provided to allow a quick placement of surgical devices. Optionally, the device settings are applied to the surgical devices depending upon which region the surgical device is located.

In FIG. 5, an example of the operating room 100 with the defined regions is shown. A first region (region A) is placed in front of the patient door 110. Specifically, the size and position of the first region is such that when opening the patient doors, if region A is free from obstructions, then the patient doors 110 will open unhindered. Similarly, a second region (region B) is provided in front of the staff door 105. The size and position of region A and B should be defined appropriately. The technician will thus not place any surgical device or obstruction in either region A or B.

A third region (region C) is defined around the surgical microscope 135. It should be noted here that the third region is defined in the operating room 100 and the surgical microscope 135 will be placed within this region. The size and/or shape of this region will be defined, at least in part, by the size and/or shape of the surgical microscope 135. Specifically, the size of the third region will be the same size or larger than the footprint of the surgical microscope 135. The size and shape of the third region may be also dependent upon the amount of space required to accommodate the swing arm upon which the microscope is mounted. In other words, it is necessary to accommodate the movement of the swing arm within the size and shape of the third region. This reduces the likelihood of another surgical device obstructing the movement of the swing arm.

The location of the third (or any) region may be defined using one or more criteria. For example, the location of the third region may be defined according to the mobility of the surgical device being placed in that location. Specifically, if the surgical device is not mobile, then the surgical device should be positioned away from thoroughfares within the operating room 100 to avoid the surgical device being a hazard.

In some instances, the surgical device may require headroom for ventilation or like. This may define the location of the third region.

Another example is that the position of the third region may be defined according to the surgical procedure being carried out. In one instance, if a craniotomy is being carried out, the surgical microscope 135 should be placed near the patient's head.

As a further example, the position of the third region may be defined according to preferences of the surgeon. In one instance, the surgeon may be left handed. Therefore, the surgeon would likely prefer the surgical devices to be placed on his or her left hand side.

Further, the location of the third region may be defined according to its proximity to a different surgical device located in a different region of the operating room 100. For example, the surgical microscope 135 may need to be located within one metre of the surgical trolley 130 so that the surgeon may easily access both the surgical microscope 135 and the surgical trolley 130. As another example, a surgical device that is sensitive to electromagnetic radiation may need to be located a certain distance from another surgical device that emits electromagnetic radiation. This information may be provided by supplier or may be regarded as best practice in the jurisdiction where the operating room 100 is located.

The location of the third region may be defined according to previous utilisations of the available space within the operating room 100. This may include previous user experiences and typical movements of the surgical staff when performing the surgical procedure. These user experiences may be extracted from previous experiences of the surgical staff performing the surgical procedure or using the same operating room 100, previous experiences of other surgical staff performing the surgical procedure or using the same operating room 100 or the like.

In embodiments, the regions may be split into two or more sub-regions. For example, a 2D centimetre-scale bay (e.g. on a table, trolley, patient covering drape etc.) for a single surgical device such as an endoscope, which when placed in such a sub-region, will have certain device settings applied thereto. Moreover, a 3D region covering one or more surgical devices, within which multiple device settings may be applied to multiple surgical devices. For example, the region may be applied to the full volume of a multi-shelf trolley, where each shelf is a sub-region. Therefore, a surgical device may be placed on each different shelf to apply different device settings.

In embodiments, where a moveable object such as a trolley or a tray located in one region may include multiple sub-regions; each sub-region having one or more surgical devices, in the event that the moveable object is moved to a different region, the device settings applied to the one or more surgical devices may change accordingly. For example, where the multi-shelf trolley is moved to a quiet zone, all the surgical devices located on the trolley may have their device settings changed to transmission off.

Of course, as would be appreciated, one or more sub-regions may have no device settings associated therewith. For example, multiple shelf trolley may have one shelf where no device settings are applied to surgical devices located on that shelf.

Other regions are defined within the operating room 100. These are noted below.

**[Table 1]**

| **Region** | **Label** | **Surgical Device** |
|---|---|---|
| First | Region A | Patient Entrance |
| Second | Region B | Staff Entrance |
| Third | Region C | Surgical Microscope |
| Fourth | Region D | Surgeon Trolley |
| Fifth | Region E | Transmission Off zone |
| Sixth | Region F | Anaesthetist Area |
| Seventh | Region G | Surgeon Area |
| Eighth | Region H | Monitor Bank |
| Ninth | Region I | Nurse Station |
| Tenth | Region J | Patient |

As will be apparent, the regions are defined based on the size and layout of the operating room, the surgical procedure being carried out and the identity of one or more members of the surgical team.

Further the region may be a sterilised or non-sterilised area. For example, in the sterilised area, a surgical device may be placed prior to entry or incision into the patient. Whilst located in the sterilised area the surgical device may still operate.

However, after use, the surgeon may place the surgical device in a non-sterilised area. After the surgical device has been placed in the non-sterilised area, the surgical device should not then be used on the patient as the device may be contaminated. In other words, the characteristic of the surgical device becomes non-sterile. Accordingly, the surgical device should be made non-operational to avoid the risk of the surgeon accidently using the possibly contaminated surgical device again.

The boundary between the sterilised and non-sterilised regions may be defined by being a predetermined distance from the surgical table. Also the boundary may be defined by the drape placed over the patient. These may be identified from an image captured of the operating room.

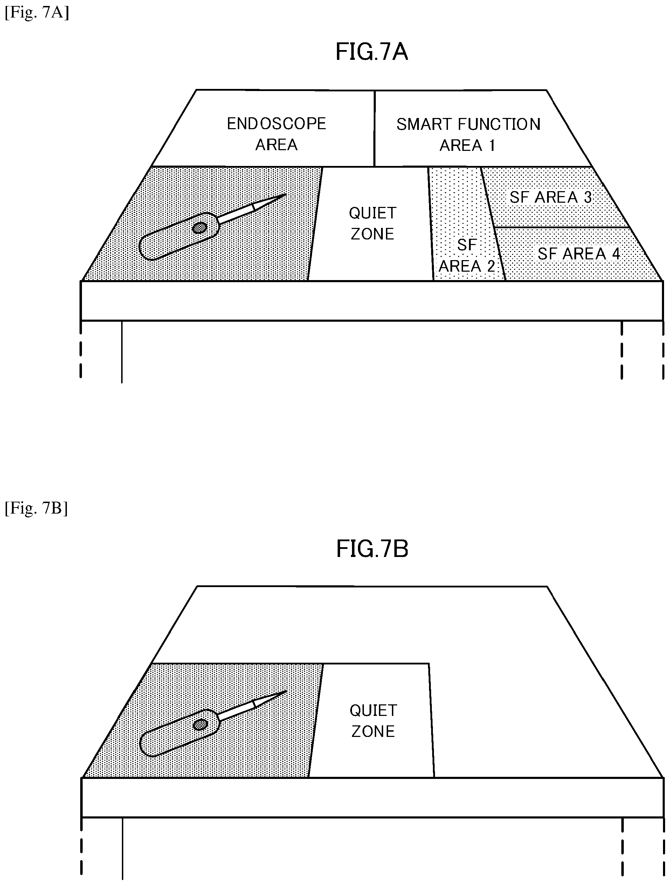

In embodiments, the regions may be provided for a part of the operating room 100 (as well as for the operating room 100 itself). For example, as shown in FIG. 7A, an operating room table is shown with various regions defined thereon. This includes an area for an endoscope, a quiet zone (the transmission off region), and areas for other various predefined functions (SF Area 1-4). This is also shown in FIG. 7B where only two regions is shown.

### <Setup>

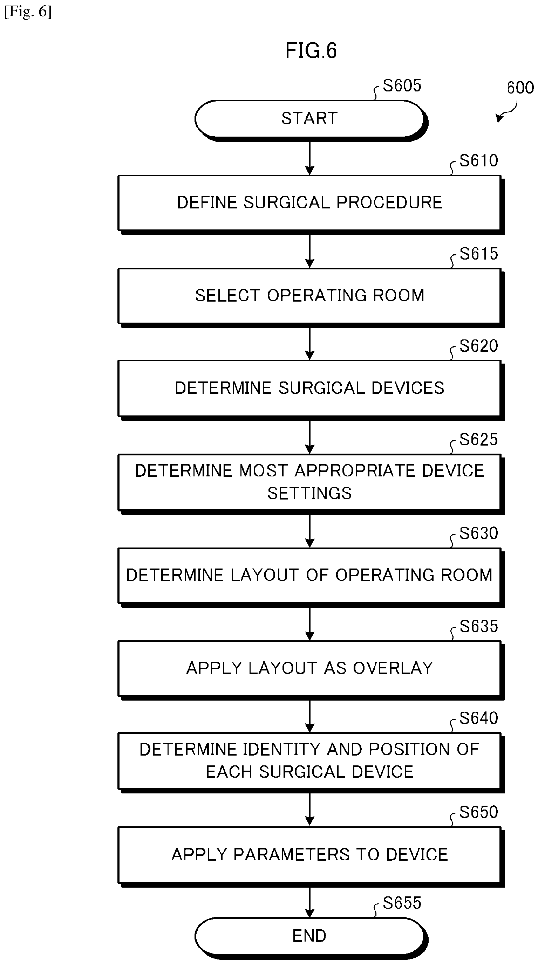

The operating room setup carried out by the room control device 200 will now be described with reference to FIG. 6.

The process 600 in FIG. 6 starts at step 605. The process then moves to step 610 where the surgical procedure, the time of the start of the surgical procedure and members of the surgical team are defined. This may be provided by the technician or may be provided to the room control device 200 by the central medical facility. Based upon the capacity of the operating room, availability of the operating room and any required infrastructure for the surgical procedure, an operating room is selected. This is step 615.

The process then moves to step 620 where the required surgical devices for the surgical procedure is determined by the room control device 200. This information would be based on surgical best practice or preferences of the surgeon for this type of procedure.

The process moves to step 625 where, optionally, the most appropriate device settings for one or more of the surgical devices are determined. This information is again based upon best surgical practice or preference of the surgeon for this type of procedure.

The process moves to step 630 where the layout of the operating room is determined. In other words, the layout and size of the regions into which the surgical devices are placed is determined. The layout of the operating room may be determined based on previous configurations stored within the room control device 200. This would take into account the position of patient entrances, the location of the patient for such a surgical procedure and preferences of the surgeon.

Of course, the disclosure is not so limited and the layout of the operating room may be automatically configured. This may be achieved using any known layout optimisation technique. In particular, the position of each region is defined using the position of the patient entry/exit, the surgical team entry/exit and patient position. Moreover, other factors such as size and orientation of the surgical device relative to the patient may be used. Of course, an existing template for the layout of an operating room for the surgical procedure may be adjusted.

The process then moves to step 635 where an overlay of the regions in the operating room 100 is provided to the technician. The regions are defined by positional information such as a set of co-ordinates or other positional information. A label is provided in each region to allow correct placement of the surgical devices in the correct region. The resolution of the region overlaid in the operating room 100 may vary with objects located in the operating room. For example, the resolution for a surgical device being located on a trolley is higher than for a surgical device being located in the operating room more generally.

The regions may be modified by person (such as the technician, or a member of the surgical team), during or prior to a procedure via a user interface. This information may update future layouts of the operating room for the surgeon or for the selected surgical procedure.

The process then moves to step 640. In step 640, the identity and position of each surgical device is determined by the room control device 200. This may be achieved using image recognition or using positional information received from each surgical device. The position of each surgical device is compared with the region layout to ensure that the correct surgical device is located within the correct region. This verification step reduces the likelihood of human error.

The process then moves to step 645. In step 645, the device parameters are applied to the surgical devices depending upon the region in which the surgical devices are located. These device parameters may be based upon the surgical procedure being carried out, the identity of the surgeon or members of the surgical team or may be default settings for the surgical device. These device parameters may be applied automatically by the room control device 200 via the surgical device communication circuitry 315, or may be applied by the technician.

It should be appreciated that the device parameters may change depending upon interactions between various surgical devices and between the surgical devices and members of the surgical team. For example, if the surgeon changes a brightness setting on an endoscope due to lighting within the operating room, the device parameter may be updated.

Further the priority level associated with a particular device at any point within the surgical procedure will change in embodiments. In particular, during a surgical procedure, immediate access to a device becomes important. So, importance of accessibility to a device may change according to the relevance of that device to an upcoming action. For example, a cauterisation device to stop bleeds may be given a high priority prior to an incision action near an artery, with a high chance of life threatening mistakes being made. This means that it is important to access the device quickly and so the device's priority increases.

Device priority may also refer to communication priority or other differential priori-tisations based on the above assessment of the different relevancies of the device functions to the current surgical context.

The process then ends in step 650.

Although the foregoing has described applying device parameters based upon the region in which the surgical device is located, the disclosure is not so limited. For example, different device parameters may be applied by the surgeon using gesture recognition techniques or the like performed by the surgeon.

Although the foregoing has been described with the room control device 200 initiating the process of FIG. 6 manually, the disclosure is not so limited. Indeed, the room control device 200 may monitor a centralised database within a medical facility to identify upcoming operations, for example via access to an operating room schedule. Other device parameters and relevant information stored in the centralised may be used in determining the room layout.

This monitoring may automatically trigger the operation of the process 600.

Obviously, numerous modifications and variations of the present disclosure are possible: within the scope as defined by the appended claims, the disclosure may be practiced otherwise than as specifically described herein.

In so far as embodiments of the disclosure have been described as being implemented, at least in part, by software-controlled data processing apparatus, it will be appreciated that a non-transitory machine-readable medium carrying such software, such as an optical disk, a magnetic disk, semiconductor memory or the like, is also considered to represent an embodiment of the present disclosure.

It will be appreciated that the above description for clarity has described embodiments with reference to different functional units, circuitry and/or processors. However, it will be apparent that any suitable distribution of functionality between different functional units, circuitry and/or processors may be used without detracting from the embodiments.

Described embodiments may be implemented in any suitable form including hardware, software, firmware or any combination of these. Described embodiments may optionally be implemented at least partly as computer software running on one or more data processors and/or digital signal processors. The elements and components of any embodiment may be physically, functionally and logically implemented in any suitable way. Indeed the functionality may be implemented in a single unit, in a plurality of units or as part of other functional units. As such, the disclosed embodiments may be implemented in a single unit or may be physically and functionally distributed between different units, circuitry and/or processors.

## Claims

1. An operating room control system, comprising circuitry configured to:
determine a plurality of regions located within at least part of the operating room, at least one of the regions having a predefined surgical device attribute defining a characteristic of a surgical device located within that region; and
control a display to display to a user the at least one region overlaid on the at least part of the operating room;
wherein the surgical device attribute comprises specific surgical device settings to be applied to a particular surgical device;
and wherein the circuitry is configured to: communicate with the surgical device to transfer the surgical device attribute when the surgical device is located within that region.

2. An operating room control system according to claim 1, wherein the circuitry is configured to:
determine the plurality of regions and the predefined surgical device attribute based upon the surgical procedure to be carried out within the operating room.

3. An operating room control system according to claim 2, wherein the circuitry is further configured to: determine the at least one region and the predefined surgical device attributes based upon preferences of the surgeon carrying out the surgical procedure to be carried out within the operating room.

4. An operating room control system according to claim 1, wherein the circuitry is further configured to: determine the predefined surgical attribute based upon the surgical procedure to be carried out within the operating room.

5. An operating room control system according to claim 1, wherein the circuitry is further configured to: detect a gesture and change the surgical device attribute based upon the detected gesture.

6. An operating room control system according to claim 1, wherein the circuitry is configured to: define the surgical device attribute of one surgical device based upon the surgical device attribute of another surgical device.

7. An operating room control system according to claim 6, wherein the defined surgical device attribute of the one surgical device is to disable a communication function of the one surgical device.

8. An operating room control system according to claim 1, wherein the circuitry is configured to: disable a communication function of the surgical device as the surgical device attribute.

9. An operating room control system according to claim 1, wherein the circuitry is further configured to set the brightness of light within the operating room in accordance with the surgical device attribute.

10. An operating room control system according to claim 1, wherein the circuitry is further configured to set the characteristic of the surgical device in dependence upon the experience of a surgeon using the surgical device.

11. An operating room control system according to claim 1, wherein the circuitry is further configured to determine a monitor configuration for the surgical device based upon the surgical device attribute.

12. An operating room control system according to claim 1, wherein the circuitry is further configured to set the imaging characteristic of the surgical device in accordance with the tissue of the patient being imaged.

13. An operating room control method, comprising:
determining a plurality of regions located within at least part of the operating room, at least one of the regions having a predefined surgical device attribute defining a characteristic of a surgical device located within that region; and
controlling a display to display to a user the at least one region overlaid on the at least part of the operating room;
wherein the surgical device attribute comprises specific surgical device settings to be applied to a particular surgical device;
and wherein the method further comprises: communicating with the surgical device to transfer the surgical device attribute when the surgical device is located within that region

14. A computer program comprising computer readable instructions which, when loaded onto a computer, configures the computer to perform a method according to claim 13.

## Patentansprüche

1. Operationssaalsteuerungssystem, das eine Schaltlogik umfasst, die konfiguriert ist zum:
Bestimmen einer Vielzahl von Regionen, die sich mindestens innerhalb eines Teils des Operationssaals befinden, wobei mindestens eine der Regionen ein vordefiniertes chirurgisches Vorrichtungsattribut aufweist, das eine Eigenschaft einer chirurgischen Vorrichtung definiert, die sich innerhalb dieser Region befindet; und
Steuern einer Anzeige, um einem Benutzer die mindestens eine Region anzuzeigen, die dem mindestens einen Teil des Operationssaals überlagert ist;
wobei das chirurgische Vorrichtungsattribut spezifische chirurgische Vorrichtungseinstellungen umfasst, die auf eine bestimmte chirurgische Vorrichtung anzuwenden sind;
und wobei die Schaltlogik konfiguriert ist zum: Kommunizieren mit der chirurgischen Vorrichtung zum Überführen des chirurgischen Vorrichtungsattributs, wenn sich die chirurgische Vorrichtung innerhalb dieser Region befindet.

2. Operationssaalsteuerungssystem nach Anspruch 1, wobei die Schaltlogik konfiguriert ist zum: Bestimmen der Vielzahl von Regionen und des vordefinierten chirurgischen Vorrichtungsattributs basierend auf der chirurgischen Prozedur, die innerhalb des Operationssaals durchgeführt werden soll.

3. Operationssaalsteuerungssystem nach Anspruch 2, wobei die Schaltlogik ferner konfiguriert ist zum: Bestimmen der mindestens einen Region und der vordefinierten chirurgischen Vorrichtungsattribute, basierend auf den Präferenzen des Chirurgen, der die chirurgische Prozedur durchführt, die in dem Operationssaal durchgeführt werden soll.

4. Operationssaalsteuerungssystem nach Anspruch 1, wobei die Schaltlogik ferner konfiguriert ist zum: Bestimmen des vordefinierten chirurgischen Attributs basierend auf der chirurgischen Prozedur, die in dem Operationssaal durchgeführt werden soll.

5. Operationssaalsteuerungssystem nach Anspruch 1, wobei die Schaltlogik ferner konfiguriert ist zum: Festellen einer Geste und Ändern des chirurgischen Vorrichtungsattributs basierend auf der festgestellten Geste.

6. Operationssaalsteuerungssystem nach Anspruch 1, wobei die Schaltlogik konfiguriert ist zum: Definieren des chirurgischen Vorrichtungsattributs einer chirurgischen Vorrichtung basierend auf dem chirurgischen Vorrichtungsattribut einer anderen chirurgischen Vorrichtung.

7. Operationssaalsteuerungssystem nach Anspruch 6, wobei das definierte chirurgische Vorrichtungsattribut der einen chirurgischen Vorrichtung dazu dient, eine Kommunikationsfunktion der einen chirurgischen Vorrichtung zu deaktivieren.

8. Operationssaalsteuerungssystem nach Anspruch 1, wobei die Schaltlogik konfiguriert ist zum: Deaktivieren einer Kommunikationsvorrichtung der chirurgischen Vorrichtung als das chirurgische Vorrichtungsattribut.

9. Operationssaalsteuerungssystem nach Anspruch 1, wobei die Schaltlogik ferner konfiguriert ist, um die Helligkeit des Lichts innerhalb des Operationssaals gemäß dem chirurgischen Vorrichtungsattribut einzustellen.

10. Operationssaalsteuerungssystem nach Anspruch 1, wobei die Schaltlogik ferner konfiguriert ist, um die Eigenschaft der chirurgischen Vorrichtung in Abhängigkeit von der Erfahrung eines Chirurgen, der die chirurgische Vorrichtung verwendet, einzustellen.

11. Operationssaalsteuerungssystem nach Anspruch 1, wobei die Schaltlogik ferner konfiguriert ist, um eine Überwachungskonfiguration für die chirurgische Vorrichtung basierend auf dem chirurgischen Vorrichtungsattribut zu bestimmen.

12. Operationssaalsteuerungssystem nach Anspruch 1, wobei die Schaltlogik ferner konfiguriert ist, um die Bildgebungseigenschaft der chirurgischen Vorrichtung gemäß dem Gewebe des abzubildenden Patienten zu setzen.

13. Operationssaalsteuerungsverfahren, umfassend:
Bestimmen einer Vielzahl von Regionen, die sich mindestens innerhalb eines Teils des Operationssaals befinden, wobei mindestens eine der Regionen ein vordefiniertes chirurgisches Vorrichtungsattribut aufweist, das eine Eigenschaft einer chirurgischen Vorrichtung definiert, die sich innerhalb dieser Region befindet; und
Steuern einer Anzeige, um einem Benutzer die mindestens eine Region anzuzeigen, die dem mindestens einen Teil des Operationssaals überlagert ist;
wobei das chirurgische Vorrichtungsattribut spezifische chirurgische Vorrichtungseinstellungen umfasst, die auf eine bestimmte chirurgische Vorrichtung anzuwenden sind;
wobei das Verfahren ferner Folgendes umfasst: Kommunizieren mit der chirurgischen Vorrichtung, um das chirurgische Vorrichtungsattribut zu überführen, wenn sich die chirurgische Vorrichtung innerhalb dieser Region befindet.

14. Computerprogramm, das computerlesbare Anweisungen umfasst, die, wenn sie auf einen Computer geladen werden, den Computer konfigurieren, um ein Verfahren nach Anspruch 13 auszuführen.

## Revendications

1. Système de commande de salle d'opération, comprenant des circuits configurés pour :
déterminer une pluralité de régions situées à l'intérieur d'au moins une partie de la salle d'opération, au moins l'une des régions ayant un attribut de dispositif chirurgical prédéfini définissant une caractéristique d'un dispositif chirurgical situé dans cette région ; et
commander un dispositif d'affichage pour afficher à un utilisateur l'au moins une région superposée sur l'au moins une partie de la salle d'opération ;
dans lequel l'attribut de dispositif chirurgical comprend des réglages de dispositif chirurgical spécifiques à appliquer à un dispositif chirurgical particulier ;
et dans lequel les circuits sont configurés pour : communiquer avec le dispositif chirurgical pour transférer l'attribut de dispositif chirurgical lorsque le dispositif chirurgical est situé dans cette région.

2. Système de commande de salle d'opération selon la revendication 1, dans lequel les circuits sont configurés pour : déterminer la pluralité de régions et l'attribut de dispositif chirurgical prédéfini sur la base de la procédure chirurgicale à effectuer dans la salle d'opération.

3. Système de commande de salle d'opération selon la revendication 2, dans lequel les circuits sont en outre configurés pour : déterminer l'au moins une région et les attributs de dispositif chirurgical prédéfinis sur la base des préférences du chirurgien effectuant la procédure chirurgicale à effectuer dans la salle d'opération.

4. Système de commande de salle d'opération selon la revendication 1, dans lequel les circuits sont en outre configurés pour : déterminer l'attribut chirurgical prédéfini sur la base de la procédure chirurgicale à effectuer dans la salle d'opération.

5. Système de commande de salle d'opération selon la revendication 1, dans lequel les circuits sont en outre configurés pour : détecter un geste et changer l'attribut de dispositif chirurgical sur la base du geste détecté.

6. Système de commande de salle d'opération selon la revendication 1, dans lequel les circuits sont configurés pour : définir l'attribut de dispositif chirurgical d'un dispositif chirurgical sur la base de l'attribut de dispositif chirurgical d'un autre dispositif chirurgical.

7. Système de commande de salle d'opération selon la revendication 6, dans lequel l'attribut de dispositif chirurgical défini du dispositif chirurgical doit désactiver une fonction de communication du dispositif chirurgical.

8. Système de commande de salle d'opération selon la revendication 1, dans lequel les circuits sont configurés pour : désactiver une fonction de communication du dispositif chirurgical en tant qu'attribut de dispositif chirurgical.

9. Système de commande de salle d'opération selon la revendication 1, dans lequel les circuits sont en outre configurés pour régler la luminosité de la lumière dans la salle d'opération conformément à l'attribut de dispositif chirurgical.

10. Système de commande de salle d'opération selon la revendication 1, dans lequel les circuits sont en outre configurés pour régler la caractéristique du dispositif chirurgical en fonction de l'expérience d'un chirurgien utilisant le dispositif chirurgical.

11. Système de commande de salle d'opération selon la revendication 1, dans lequel les circuits sont en outre configurés pour déterminer une configuration de surveillance pour le dispositif chirurgical sur la base de l'attribut de dispositif chirurgical.

12. Système de commande de salle d'opération selon la revendication 1, dans lequel les circuits sont en outre configurés pour régler la caractéristique d'imagerie du dispositif chirurgical conformément au tissu du patient qui est imagé.

13. Procédé de commande de salle d'opération, comprenant :
la détermination d'une pluralité de régions situées dans au moins une partie de la salle d'opération, au moins l'une parmi les régions ayant un attribut de dispositif chirurgical prédéfini définissant une caractéristique d'un dispositif chirurgical situé dans cette région ; et
la commande d'un dispositif d'affichage pour afficher à un utilisateur l'au moins une région superposée sur l'au moins une partie de la salle d'opération ;
dans lequel l'attribut de dispositif chirurgical comprend des réglages de dispositif chirurgical spécifiques à appliquer à un dispositif chirurgical particulier ;
et dans lequel le procédé comprend en outre : la communication avec le dispositif chirurgical pour transférer l'attribut de dispositif chirurgical lorsque le dispositif chirurgical est situé dans cette région

14. Programme informatique comprenant des instructions lisibles par ordinateur qui, lorsqu'elles sont chargées sur un ordinateur, configurent l'ordinateur pour réaliser un procédé selon la revendication 13.
